Europäisches Patentamt

European Patent Office

Office européen des brevets

(11) Veröffentlichungsnummer: **0 068 192**
**B1**

(12) **EUROPÄISCHE PATENTSCHRIFT**

(45) Veröffentlichungstag der Patentschrift:
13.08.86

(21) Anmeldenummer: 82105003.6

(22) Anmeldetag: 08.06.82

(51) Int. Cl.⁴: **B 01 J 37/00,** B 01 J 27/18, B 01 J 23/30, C 07 C 47/22, C 07 C 51/235, C 07 C 47/052, C 07 C 120/14

(54) Verfahren zur Herstellung von abriebfesten Schalenkatalysatoren und Verwendung dieser.

(30) Priorität: 26.06.81 DE 3125062

(43) Veröffentlichungstag der Anmeldung:
05.01.83 Patentblatt 83/1

(45) Bekanntmachung des Hinweises auf die Patenterteilung:
13.08.86 Patentblatt 86/33

(84) Benannte Vertragsstaaten:
BE CH DE FR GB IT LI NL

(56) Entgegenhaltungen:
EP-A-0 015 569
EP-A-0 068 193
DE-A-2 135 620
DE-A-2 805 801
FR-A-2 274 597
GB-A-666 529
GB-A-977 755
US-A-3 761 424
US-A-4 034 060

(73) Patentinhaber: Degussa Aktiengesellschaft,
Weissfrauenstrasse 9, D-6000 Frankfurt am Main
1 (DE)

(72) Erfinder: Arntz, Dietrich, Dr., Ameliastrasse 16,
D-6450 Hanau (DE)
Erfinder: Prescher, Günter, Dr., Liesingstrasse 2,
D-6450 Hanau 9 (DE)
Erfinder: Manner, Reinhard, Dr., Bonhoeffer
Strasse 17, D-6457 Maintal 1 (DE)
Erfinder: Heilos, Johannes, Westring 16, D-6453
Seligenstadt (DE)
Erfinder: Burkhardt, Werner, Reichenbachstrasse 9,
D-6486 Brachttal 11 (DE)

## Beschreibung

Die Erfindung betrifft ein Verfahren zur Herstellung von abriebfesten Schalenkatalysatoren aus einem oberflächlich rauhen, inerten Träger mit einer Korngröße von 0,5 bis 6 mm und einer diesen umhüllenden und in ihm verankerten Schale aus aktivem Katalysatormaterial durch mechanisch bewirktes mischendes Bewegen einer Schüttung des Trägers und Aufsprühen einer Suspension des Ausgangsmaterials für die Schale unter Teilabzug des Suspensionsmittels durch einen Gasstrom von 20-250 C, wobei eine im wesentlichen gleichbleibende Rest feuchte der Schale aufrechterhalten wird sowie Trocknen und Tempern. Sie betrifft ferner Verwendungen dieser Katalysatoren.

Es ist bekannt, für katalytische Oxidationen Katalysatoren zu verwenden, bei denen die katalytisch aktiven Bestandteile als Schale auf einem inerten Träger, der als Korn oder Formling vorliegt, angeordnet ist.

Durch diese Maßnahme wird pro Reaktionsvolumen weniger vom teuren katalytisch aktiven Katalysatormaterial benötigt, wodurch preiswertere Katalysatoren herstellbar sind.

Daneben können die katalytischen Eigenschaften von Katalysatoren verbessert werden. Durch diese Anordnung der aktiven Substanz an der Trägeroberfläche werden nämlich einerseits durch die temperaturausgleichende Wirkung der Trägermasse lokale Überhitzungen vermieden und andererseits die Diffusionswege für gasförmige Reaktanten verkürzt. Außerdem ist es dadurch möglich, durch Aufbringen von verschiedenen Schichtdicken in gezielter Weise mehr oder weniger aktive Katalysatoren zu erzeugen (AT-B 22 66 72).

In der DE-B 21 65 335 ist ein Verfahren zur Herstellung von Acrolein durch Oxidation von Propylen mit einem molekularen Sauerstoff enthaltenden Gas in der Gasphase bei erhöhter Temperatur in Gegenwart eines Katalysators beschrieben, bei dem z. B. eine die Elemente MoBiCoNiFeB-NaSnSiO enthaltende pulverförmige Masse auf einen inaktiven, porösen Trägerformling, wie $\alpha$-Al$_2$O$_3$, aufgezogen ist. Letzteres erfolgt hier durch Aufbringen der feuchtgemahlenen Katalysatormasse auf poröse $\alpha$-Al$_2$O$_3$-Kugeln von 5 mm Durchmesser sowie Trocknen und Tempern.

Die DE-A 23 51 151 beschreibt ein Verfahren zur Herstellung eines zur Oxidation, Ammonoxidation oder oxidativen Dehydrierung eines Olefins bestimmten Schalenkatalysators, zu dessen Bereitung ein inerter Träger mit einem Durchmesser von mindestens 20 µm mit einer Flüssigkeit vorbenetzt wird und dieser sodann mit trockenem pulverförmigem Katalysatormaterial versetzt und langsam gerührt wird.

Nach der DE-A 22 50 200 kann ein Schalenkatalysator für die Reinigung der Abgase von Kraftfahrzeugen und Industrieanlagen dadurch erhalten werden, daß man auf Formlingen aus temperaturbeständigem Trägermaterial durch intensives Vermengen mit einer vorzugsweise Teilchenabmessungen unter 100 um aufweisenden pulverförmigen calcinierten Aktivkomponente unter Verwendung eines flüssigen Bindemittels einen katalytischen aktiven Überzug erzeugt und dann das Bindemittel durch Erhitzen entfernt. Danach liegen der Trägerkern und der haftfeste Überzug ohne wesentliche gegenseitige Durchdringung vor.

Schließlich sieht das in der EP-A 0 015 569 beschriebene Verfahren zur Herstellung von Schalenkatalysatoren die Aufbringung einer wässrigen Suspension des katalytisch aktiven Materials auf bewegte Trägerteilchen vor, wobei die Suspension in einer bestimmten gleichbleibenden Menge unter Teilabzug des Suspensionsmittels vermittels eines Gasstroms von 20 - 300° C auf den Träger aufgesprüht wird und wobei eine im wesentlichen gleichbleibende Restfeuchte der Schale aufrechterhalten wird.

Den nach den bekannten Verfahren erhältlichen Katalysatoren ist der Nachteil gemeinsam, daß bei dickeren Schalen, also Schalen, deren Gewichtsmenge, bezogen auf den Katalysator, 20 % übersteigt, die Abrieb- und Stoßfestigkeit der Schale für den Einsatz in großtechnischen Festbettreaktoren nicht vollständig befriedigt.

Insbesondere zeigte sich bei Schalenkatalysatoren, welche mit konventionellen Dragierkesseln oder Drehtellern gefertigt werden, welche nur ein Überleiten eines trocknenden Gasstroms über das bewegte Gut gestatten, eine Neigung zum Abplatzen der Schale bei Einwirkung von Temperaturgradienten.

Ferner kann mit solchen Vorrichtungen nur eine relativ breite Kornverteilung, die von der jeweiligen Schalendicke der Einzelpartikel des Katalysators bestimmt wird, erzielt werden.

Eine breite Kornverteilung bedeutet aber einerseits einen deutlich höheren Druckabfall von Katalysatorschüttungen und andererseits das Auftreten stark unterschiedlicher Wärmetönungen an den einzelnen Katalysatorpartikeln, was insgesamt zu einer Verschlechterung der Selektivität führt.

Das in der EP-A 0 015 569 behandelte Herstellungsverfahren fordert die Aufrechterhaltung einer zeitlich konstantbleibenden Dosiergeschwindigkeit für Suspension und Trocknungsgas, um den Wassergehalt der entstehenden Schale während des Aufsprühens der Suspension praktisch konstant zu halten. Allerdings führt gerade diese Maßnahme dazu, daß mit zunehmender Dauer des Herstellungsganges die Äußere Oberfläche der Schale zunehmend flüssigkeitsärmer wird, was das Auftragen dickerer Schichten mit ausreichender mechanischer Festigkeit erschwert oder verhindert.

Im übrigen wird durch die dort vorgesehene Führung des Trockengasstroms über die Oberfläche der Trägerschüttung hinweg nur eine mäßige Trocknungsgeschwindigkeit während der Ausbildung der Schale erreicht. Die Folge davon ist die bereits erwähnte nachteilige breite Kornverteilung.

Der Erfindung liegt die Aufgabe zugrunde, ein Verfahren zur Herstellung von Schalenkatalysatoren zu schaffen, welches diesen Abriebfestigkeit, Temperaturwechselbeständigkeit und ein enges Kornspektrum bei gleichzeitig guten katalytischen Eigenschaften verleiht.

Gegenstand der Erfindung ist ein Verfahren zur Herstellung von abriebfesten Schalenkatalysatoren aus einem oberflächlich rauhen, inerten Träger mit einer Korngröße von 0,5 bis 6 mm und einer diesen umhüllenden und in ihmverankerten Schale aus aktivem Katalysatormaterial durch mechanisch bewirktes mischendes Bewegen einer Schüttung des Trägers und Aufsprühen einer Suspension des Ausgangsmaterials für die Schale unter Teilab, zug des Suspensionsmittels durch einen Gasstrom von 20-250° C, wobei eine im wesentlichen gleichbleibende Restfeuchte der Schale aufrechterhalten wird sowie Trocknen und Tempern. Das Verfahren ist dadurch gekennzeichnet, daß man die bewegte Trägerschüttung gleichzeitig durch Einblasen eines fluidisierenden, die Vermischung verstärkenden Gasstromes von unten her auflockert, dieser Schüttung im Gegenstrom zum Gas die ein Bindemittel und gegebenenfalls Porenbildner enthaltende Suspension einer Vorstufe des katalytisch aktiven Materials in einer mit anwachsender Oicke der Schale zunehmeden Menge zuführt wobei die Mengen von abgezogenem und aufgesprühtem Suspensionsmittel in einem weitgehend konstanten Verhältnis gehalten werden, das von der jeweils verwendeten Kombination aus Träger und Vorstufe bestimmt wird und wobei die Wärmeausdehnungskoeffizienten von Träger und getrockneter pulverförmiger Vorstufe so abgestimmt sind, daß sie sich im Höchstfall um 15 % unterscheiden, daß man nach Beendigung des Aufsprühens die Schale durch Fortsetzen der verstärkten Mischbewegung verdichtet, dann die mechanische Mischbewegung einstellt, das Gut im weiterströmenden Gas trocknet und es schließlich, gegebenenfalls nach Zersetzen eines zugegebenen Porenbildners, tempert.

Das neue Beschichtungsverfahren für Trägerkörper sieht vor, eine in Mischbewegung versetzte Trägerschüttung durch Einblasen eines Gasstromes von unten her aufzulockern, wobei der die fluidisierte Charge durchstreichende Gasstrom einen Teilabzug des Suspenisonsmittels bewirkt. Zur Durchführung kommen entsprechend eingerichtete Mischaggregate in Betracht, wie z. B. spezielle Dragiertrommeln, Dragierkessel oder Drehteller. Bevorzugt sind Apparate, in denen eine gleichmäMige Durchströmung der Trocknungsluft durch die gesamte Schüttung erfolgt.

Als besonders günstig hat sich erwiesen, das Aufbringen und Trocknen der Schale in einer Vorrichtung durchzuführen, die primär aus einer zylindrisch bzw. kegelig geformten und horizontal montierten Trommel besteht. indem über im Außenmantel der Trommel angebrachte Luftkanäle und über auf der Trommelinnenwand angeordnete, auf der Drehrichtungs-abgekehrten Seite perforierte Hohlrippen Trockenluft ausschließlich von der Gutbett-Unterseite her eingebracht wird.

Ein solches Gerät, bei welchem Sprühflüssigkeit und Trockenluft in entgegengesetzter Richtung strömen, ist der u. a. in der DE-A 28 05 801 beschriebene sog. Driacoater.

Beim Drehen der Trommel dieses Geräts bewirken die wulstförmig gestalteten Hohlrippen und die durch diese eingeblasene Trocknungsluft die Fluidisierung und intensive Umwälzung des Schüttgutes; dessen gleichmäßige Durchströmung durch die Trocknungsluft zeigt sich in einer gleichförmig ruhig abwärtsfließenden Eigenbewegung des Gutes. Die mit Feuchtigkeit beladene Abluft wird oberhalb der Schüttung über den hohlen Aufnahmedorn in der Drehachse der Trommel abgezogen.

Für das Versprühen der im erfindungsgemäßen Verfahren verwendeten Pulversuspensionen werden bevorzugt Zweistoffdüsen verwendet, mit denen einfacher als bei Einstoffdüsen die gewünschte Fördermenge unter beliebiger Feinverteilung bequem reguliert werden kann.

Die Zerstäubung erfolgt üblicherweise mit Preßluft von 0,5 - 2 bar und in Abhängigkeit vom notwendigen Suspensionsdurchsatz (der sich aus der Chargengröße, der gewünschten Dicke des Pulverauftrages und der Präparationsdauer ergibt) mit ein oder mehreren Düsen von 2 - 4 mm Durchmesser bei Drucken der Suspension vor der Düse von 1 - 3 bar.

Bei Driacoater-Geräten mit Chargenkapazitäten von 10 - 200 l hat es sich als zweckmäßig erwiesen, den fluidisierenden Gasstrom auf einen spezifischen Durchsatz von 15-50 Nm$^3$/Stunde/l Träger einzustellen und ihn auf 60 - 100° C aufzuheizen. Geringere Zuluftdurchsätze führen zu deutlich langsameren Trockengeschwindigkeiten, weniger gleichmäßiger Durchströmung der gesamten Schüttung durch Randgängigkeit an der Trommelwandung und damit wesentlich längeren Präparationszeiten. Zu hohe Zuluftdurchsätze verursachen dagegen ein zu starkes Austrocknen der Suspension auf dem Weg von der Düse zur Schüttungsoberfläche, wodurch ein Austrag von dabei ausgetrocknetem Vorstufen-Pulver mit der Abluft und eine nicht ausreichende Feuchte der Schale beim Auftrag verursacht wird. Es wurde gefunden, daß das Einhalten einer konstanten Feuchte der entstehenden Schale während des gesamten Schalenaufbaus eine wesentliche Voraussetzung zur Erzielung eines festhaftenden, fest im Trägermaterial verankerten Überzuges von aktivem Katalysatormaterial ist. Wird die Schale der Rohlinge während dieses Schalenaufbaus zu feucht, so erfolgt ein Agglomerieren mehrerer Körner miteinander. Bei zu trockenem Auftrag ist dagegen nicht die erwünschte Verankerung am Träger und auch keine gute Festigkeit der Schale zu erreichen. Eine wesentliche Erkenntnis besteht auch darin, daß sich bei Aufrechterhaltung einer bezüglich Temperatur und Menge konstanten Trocknungszuluft die erforderliche konstante Feuchte der Schale über die pro Zeiteinheit aufgesprühte Menge Suspension gut steuern läßt.

Zur Sollwertvorgabe für eine solche Steuerung kann die Temperatur über der Schüttung oder die Feuchte der Abluft verwendet werden, die beide eine sensible Verfolgung des Trocknungsvorganges erlauben. Die günstigsten Sollwerte selbst sind abhängig von der Art des Pulvers und der Temperatur, der Feuchte und der Henge der Zuluft pro Volumeneinheit Trägermaterial. Je nach Feststoffgehalt der Suspension und Art der Vorstufe sollten 10 - 50 % des aufgesprühten Suspensionsmittels während deren Aufbau in der Schale bleiben. Es wurde gefunden, daß eine wesentliche Verbesserung der mechanischen Stabilität der Schale erreicht wird, wenn kein konstanter Sollwert, sondern eine fallende Temperatur bzw. eine steigende Abluftfeuchte vorgegeben wird. Dies ermöglicht durch eine entsprechende Programmsteuerung einen vollautomatischen Auftrag des Vorstufenpulvers.

Als Suspensionsmittel für die in Pulverform vorliegende Katalysatorvorstufe wird bevorzugt Wasser verwendet. Andere Flüssigkeiten wie z. B. Alkohole, sind nicht ausgeschlossen und haben in verschiedenen Punkten gegenüber Wasser Vorteile: Sie benötigen gegebenenfalls geringere Verdampfungsenergie oder erlauben eine bessere Anpassung von Benetzungs- und Löslichkeitsverhalten an die Vorstufe des katalytischen Materials und die Trägersubstanz. Letzteres ist bei wässrigen Suspensionen nur durch Zugabe von Bindemitteln beeinflußbar. Dem Vorteil organischer Lösungsmittel steht jedoch der Nachteil entgegen, mit der Trocknungsluft zündfähige Gemische zu bilden und spezielle Abluftreinigunganlagen zu erfordern.

Der Festkörpergehalt der Suspension wird am besten so bemessen, daß die Suspension zu 20 - 80, vorzugsweise 40-70 Gew.%, insbesondere 55 Gew.% aus pulverförmiger Vorstufe besteht.

Zu hohe Feststoffgehalte können Verstopfungen des Förderund Sprühsystems für die Suspension verursachen. Zu geringe Feststoffgehalte erfordern dagegen unnötig verlängerte Präparationszeiten. Der empirisch ermittelbare, jeweils günstigste Feststoffgehalt hängt von den Eigenschaften der eingesetzten Vorstufe und ihrer Wechselwirkung mit dem Suspensionsmittel ab und liegt z. B. bei den im Rahmen der Beispiele für die Propenoxidation hergestellten Katalysatoren bei 55 %.

Es hat sich weiter gezeigt, daß eine deutliche Verbesserung der Abriebfestigkeit der Trägerkontakte durch die Verwendung von Bindemitteln, wie sie aus der Granulation bekannt sind, erreicht werden kann. Ihr Anteil an der Suspension ist abhängig von der Art des Bindemittels und liegt in der Regel zwischen 0,5 und ID %. Während die untere Grenze fließend ist und bei der minimal notwendigen Menge zur gesicherten Verbesserung der Abriebfestigkeit liegt, wird bei zu hohen Bindemittelkanzentrationen die Trocknungsgeschwindigkeit während der Herstellung der Schale häufig verringert. Für die verwendeten Vorstufen der aktiven Katalysatorkomponente wurden die besten Ergebnisse mit 2 - 5 %, insbesondere ca. 4 Gew.% Glucose oder Harnstoff erreicht.

Bei bestimmten Reaktionen, wie z. B. der Oxidation von Propen zu Acrolein, wird insbesondere bei Verwendung von Schalenkatalysatoren mit hohem Anteil an aktiver Phase, d. h. dicken Schalen, eine auf Porendiffusion zurückgehende Hemmung der Reaktion beobachtet. Es wurde nun gefunden, daß durch Zugabe von im Suspensionsmittel schwerlöslichen, feinteiligen Porenbildnern, wie Pentaerithrit, Polymethylmethacrylat, Polystyrol, Polyvinylalkohole o. Ä., dieser hemmende Einfluß auf die Reaktion durch Ausbildung von Makroporen verringert werden kann.

Der bevorzugte Gehalt der Suspension an Porenbildner beträgt 1 - 10 Gew.%. Voraussetzung für die Wirkung des Porenbildners ist, daß er unterhalb der Tempertemperatur durch Thermolyse oder Oxidation aus der aufgebauten Schale wieder entfernt werden kann.

Die Erfindung sieht ausdrücklich vor, für den Schalenaufbau eine Vorstufe des katalytisch aktiven Materials heranzuziehen. Der Begriff "Vorstufe" ist so zu verstehen, daß das Vorstufenmaterial bereits alle Ingredienzien enthält, die für die Erzeugung des fertigen katalytisch aktiven Materials durch eine anschließende spezifische Wärmebehandlung benötigt werden.

Beim Verfahren gemäß Erfindung wird bevorzugt als Vorstufe des katalytisch aktiven Materials ein getrocknetes oder unterhalb der Tempertemperatur calciniertes Kopräzipitat aus vereinigten Salzlösungen der katalytisch aktiven Elemente eingesetzt.

Die Zusammensetzung dieses Kopräzipitats und seine spezielle Präparation ist nicht spezifisch für das erfindungsgemäße Verfahren, sondern abhängig von der gewünschten katalytischen Wirkung in der Reaktion, bei der der Schalenkatalysator eingesetzt wird. Üblicherweise kann die Präparation der Vorstufe analog der von bekannten Vollkatalysatoren erfolgen. Zur Erzielung einer guten Suspendierbarkeit der Vorstufe in dem Suspensionsmittel und einer störungsfreien Förderung der Suspension hat sich eine Kornverteilung von 1 - 150 μm mit einem vorzugsweisen Maximum im Bereich von 1,5.- 30 μm als zweckmäßig erwiesen.

Das erfindungsgemäße Verfahren ermöglicht die Fertigung von Schalenkatalysatoren, bei denen die Menge der pulverförmigen Vorstufe das 0,1 - 2-fache des Trägergewichts beträgt, wobei sich dieser Bereich nicht durch spezifische Grenzen des Herstellungsverfahrens, sondern mehr aus praktischen Überlegungen zum Einsatz der erfindungsgemäßen Katalysatoren ergibt. Dies bedeutet, daß grundsätzlich auch Zusammensetzungen außerhalb des angegebenen Bereichs nach dem erfindungsgemäßen Verfahren herstellbar sind.

Die Erfindung sieht ferner ausdrücklich vor, die Wärmeausdehnungskoeffizienten von Träger und Vorstufe so abzustimmen, daß sie weitgehend übereinstimmen und sich im Höchstfall um nicht mehr als 15 % unterscheiden. Weichen nämlich diese Koeffizienten stärker voneinander ab, so reißt beim abschließenden Temperungsschritt die Schale.

Diese Risse können so groß werden, daß es zu einem schuppigen Abplatzen der Schale kommt. In jedem Fall ist mit dem Auftreten von Rissen eine starke Verringerung der mechanischen Stabilität der Schale, d. h. der Abriebfestigkeit, verbunden. Es wurde gefunden, daß eine Anpassung der Wärmeausdehnungskoeffizienten durch Auswahl eines geeigneten Trägers nur bedingt möglich ist und selten ausreicht, da die in Frage kommenden inerten Träger alle in dem relativ engen Bereich von 50 - 90. $10^{-7}/°$ C (für eindimensionale Ausdehnung) liegen.

Überraschend war nun die Feststellung, daß sich der Wärmeausdehnungskoeffizient des Vorstufenpulvers durch eine Temperaturvorbehandlung bei 250 - 600° C auf den des Trägers abstimmen läßt. Die jeweils genauen Bedingungen hänge von der Zusammensetzung der Vorstufe und dem zu verwendenden Träger ab. Dabei ist darauf zu achten, daß diese Anpassung nicht für eine bestimmte Temperatur, sondern für den gesamten Temperaturbereich der abschließenden Temperung erfolgen muß (die bei dieser Temperung auftretenden Spannungen zwischen Schale und Träger sind für die eventuelle Rißbildung verantwortlich). Dadurch ist eine exakte Abstimmung, die eine fest definierte Bezugstemperatur voraussetzen würde, nicht möglich. Dies beruht insbesondere auf dem Umstand, daß bei den erfindungsgemäß anzuwendenden Materialien üblicherweise unterschiedliche Temperaturabhängigkeiten der Ausdehnungskoeffizienten für Vorstufe und Träger vorliegen.

Im Rahmen der Erfindung soll die Bereitung der verbesserten Schalenkatalysatoren für vier wichtige Gasreaktionen der organischen Chemie, welche mit heterogener Katalyse arbeiten, besonders herausgestellt werden, weil gerade diese Verfahren mit diesen Katalysatoren erheblich verbessert werden können.

Es ist dies die in der EP-B-0 068 193 beschriebene katalytische Oxidation von Propylen oder Isobuten zu Acrolein bzw. Methacrolein, die katalytische Gasphasenoxidation von Acrolein bzw. Methacrolein zu Acrylsäure bzw. Methacrylsäure, die katalytische Gasphasenoxidation von Methanol zu Formaldehyd und die Ammonoxidation von aliphatischen und aromatischen Kohlenwasserstoffen zu Nitrilen. Für die drei letztgenannten Reaktionen wird nachfolgend jeweils die Herstellung eines geeigneten Schalenkatalysators mit einem an sich bereits bekannten Vorstufenmaterial, welches nur noch der abschließenden, die katalytischen Eigenschaften prägenden Temperung bedarf, angegeben.

Nach einer bevorzugten Ausführungsform der Erfindung wird als Vorstufe für einen Schalenkatalysator zur Herstellung von Acrylsäure bzw. Methacrylsäure aus Acrolein bzw. Methacrolein ein oxidisches Pulver der Zusammensetzung $Sb_{1-60}Mo_{12}V_{0.5-25}W_{0.1-12}M_{0-12}O_x$, wobei M mindestens eines der Elemente Blei, Silber, Kupfer, Zinn, Titan, Wismut bedeutet, eingesetzt und der Schalenkatalysator 0,05 - 5 Stunden bei 320 - 450° C getempert.

Daneben kann als Vorstufe für einen Schalenkatalysator zur Oxidation von Methanol zu Formaldehyd mit Vorteil ein oxidisches Pulver aus Molybdän und Eisen, mit einem $MoO_3 : Fe_2O_3$-Verhältnis von 10, dem gegebenenfalls 3 - 60 Gew.% $TiO_2$ beigemengt sein können, eingesetzt und der Schalenkatalysator 3 - 10 Stunden bei 300 - 500° C getempert werden.

Schließlich läßt sich als Vorstufe zu einem Schalenkatalysator für die Ammonoxidation von alkylsubstituierten aromatischen und heteroaromatischen Kohlenwasserstoffen ein oxidisches Pulver aus Antimonoxid und Vanadiumoxid im Verhältnis 1,1 : 1 bis 50 : 1 einsetzen, das zusätzlich wenigstens eines der Elemente Eisen, Kupfer, Titan, Kobalt, Mangan und Nickel und gegebenenfalls Trägersubstanz aus Schichtensilikat und hochdispersem Siliciumdioxid enthält, wobei der erhaltene Schalenkatalysator bei 2 - 8 Stunden zwischen 600 und 1.100° C getempert wird.

Als Trägermaterialien für erfindungsgemäß erhältliche Schalenkatalysatoren, die sich in diesen, aber auch anderen Umsetzungen vorteilhaft einsetzen lassen, haben sich insbesondere α-Aluminiumoxid, Aluminiumsilikat, Magnesiumsilikat oder Siliciumcarbid erwiesen. An die Form der Träger werden keine speziellen Anforderungen durch das Verfahren gestellt, jedoch werden kugelförmige bevorzugt.

Porenfreies oder porenarmes Magnesiumsilikat oder Siliciumcarbid werden vor allem verwendet, wenn die aktive Phase nur oberflächlich auf den Träger aufgebracht und nicht in Hohlräumen des Trägers eingelagert werden soll. Das katalytische Material ist dagegen in den Hohlräumen von makroporösen α-Aluminiumoxiden und Alumosilikaten stärker geschützt, besser verankert und erfordert bei nicht zu starken Beschichtungen (kleiner 20 Gew.% aktive Phase) keine so harte Schale. Die Makroporen von Aluminiumsilikaten und α-Aluminiumoxid sollten im Bereich von 2 - 2000, vorzugsweise 20 - 300 μm (90 %-Wert) liegen, um einerseits eine ausreichende Festigkeit des Trägers zu gewährleisten, andererseits aber das Ablagern der aktiven Phase in den Poren zu erlauben.

Vom Gesichtspunkt eines günstigen Verhaltens beim Schalenaufbau ergeben sich für die porenarmen bzw. -freien Träger Vorteile, da bei diesen zu Anfang der Präparation eine geringere Flüssigkeitsbeladung des Trägers auftritt und die bei makroporösen Trägern am Ende der Präparation aus den Poren austretende Feuchtigkeit beim Trocknungsprozeß schwieriger zu beherrschen ist.

Die Erfindung sieht auch vor, daß das Trägermaterial eine rauhe Äußere Oberfläche haben soll, weil damit die Haftfestigkeit der Schale durch eine Tiefenverankerung des katalytisch aktiven Materials im Träger erhöht und ein gleichmäßiger Auftrag auf der gesamten Trägeroberfläche ermöglicht wird. Bei glatten Trägermaterialoberflächen wird meist ein schuppiger, unregelmäßiger dicker Auftrag beobachtet. Als besonders günstig hat sich gezeigt, wenn die Trägeroberfläche eine Rauhigkeit, charakterisiert durch den Mittenrauhwert nach DIN 4768/1, gemessen mit dem Rauhtiefenmesser nach Hommel, von 5 - 50μm aufweist.

Weitere Gegenstände der Erfindung sind neben den beschriebenen verbesserten Herstellungsverfahren für Schalenkatalysatoren die drei bereits genannten und in den Patentansprüchen 19-22 aufgenommenen Verwendungen derselben.

Die Erfindung wird anschließend anhand von Ausführungsbeispielen weiter erläutert:

**Beispiel 1**

Das Kopräzipitat für die Herstellung der aktiven Katalysatorphase wird in der aus der DE-C 20 49 583 bekannten Weise bereitet, indem einer Lösung von 32,3 kg Nickelnitrat $Ni(NO_3)_2 \cdot 6H_2O$ 1 kg Kobaltnitrat $Co(NO_3)_2 \cdot 6H_2O$ und 4,5 kg Eisennitrat $Fe(NO_3)_3 \cdot 9H_2O$ in 38 kg Wasser nacheinander eine Lösung von 0,3 kg Samariumoxid $Sm_2O_3$ in 3,5 kg 38 %iger Salpetersäure, 5,8 kg Aerosil 200®, 10.8 kg Montmorillonit, eine Lösung von 23,4 kg Ammonmolybdat $(NH_4)_6Mo_7O_{24} \cdot 4H_2O$ in 31,4 kg 3,5 %iger Phosphorsäure und eine Lösung von 5,4 kg Bismutnitrat $Bi(NO_3)_3 \cdot 5H_2O$ in 4,5 kg 7,7 %iger Salpetersäure unter Rühren zugesetzt wird. Die erhaltene Suspension des Kopräzipitats wird auf einem Walzentrockner getrocknet, bei 530° C im Drehrohr calciniert und dann gemahlen. Das so erhaltene Pulver der Vorstufe des katalytisch aktiven Materials hat eine Kornverteilung von 2 - 40μm (> 90 %, Maximum bei 15 μm) und bei 400° C einen Wärmeausdehnungskoeffizienten von $81.10^{-7}/°$ C.

Durch Aufschlämmen von 6,5 kg dieses Vorstufenpulvers in 4,7 kg Wasser unter Zusatz von 0,5 kg D-Glucose als Bindemittel und 0,3 kg Pentaerithrit (Typ R, Fa. Degussa) als Porenbildner wird die Suspension für das Ausgangsmaterial der Schale hergestellt. Als Träger zu diesem Vorstufenmaterial werden gebrannte Steatit-Kugeln mit 4 mm Durchmesser ausgewählt, die praktisch porenfrei sind, eine rauhe Oberfläche

(Mittenrauhwert 25 μm nach DIN 4768/1) haben und deren longitudinaler Wärmeausdehnungskoeffizient bei 400° C bei 90. $10^{-7}/°$ C liegt.

6 kg des Trägers werden in einen Driacoater 500 gegeben und in diesem durch Einblasen von 2 $m^3$/Min. auf 80° C vorgeheizter Luft und Drehen der Trommel mit 20 Umdrehungen pro Minute in intensive Misch- und Fließbewegung versetzt. Auf den so bewegten Träger werden zunächst in 2 Minuten 0,4 l der Suspension mit einer Zweistoffdüse aufgesprüht.

Das Aufsprühen der restlichen Suspension wird so über die Ablufttemperatur aus dem Kessel gesteuert, daß ständig eine gleichbleibende Feuchte der Schale beobachtet wird. Dabei fällt die Ablufttemperatur von anfänglich 48° C auf 39° C am Ende des Suspensionsauftrags (nach 60 Min.) ab und der Suspensionsauftrag steigt von 0,096 auf 0,104 l/ Min.

Nach Ende des Aufsprühvorgangs schließt sich bei weiterdrehender Trommel eine Verdichtungsphase von 5 Minuten und dann eine 20-minutige Trockenphase bei nur einmaliger Kesselumdrehung pro Minute an.

Nach Lufttrocknen über Nacht wird der Porenbildner im Drehrohr bei 400° C und einer mittleren Verweilzeit von 15 Minuten zersetzt. Die Aktivierung des Katalysators erfolgt bei 550° und 15 Minuten Verweilzeit ebenfalls im Drehrohr.

Der erhaltene Schalenkatalysator hat eine harte, rißfreie Schale. Der mittlere Durchmesser der erhaltenen Schalenkatalysatoren beträgt 5,25 mm mit einer Standardabweichung von 0,3 mm. Der Abrieb wird im La-Roche-Friabilator durch Roll- und Fallverschleiß bei 20 Umdrehungen pro Minute als Abrieb von kleiner 2 mm nach 7 Minuten bestimmt. Er betrug für den getemperten Schalenkatalysator weniger als 0,2 Gew.%. Nach einer Temperaturbehandlung von 100 Zyklen Aufheizen und Abkühlen, in denen der Katalysator innerhalb von 0,5 Stunden jeweils von 250° C auf 400° C aufgeheizt und wieder auf 250° C abgekühlt wurde, war der Wert nicht signifikant erhöht und lag bei 0,2 Gew.%.

Beim Falltest, freier Fall von 100 ml Kontakt durch ein 3,4 m langes Rohr mit 20 mm Innendurchmesser auf eine harte Unterlage, entstand ein Anteil an Bruch von < 2 mm von 0,03 Gew.%.

**Vergleichsbeispiel 1**

Das Vorstufenpulver wurde wie im Beispiel 1 hergestellt, nur mit dem Unterschied, daß das walzengetrocknete Kopräzipitat bei 410° C im Drehrohr calciniert wurde. Der Wärmeausdehnungskoeffizient des Pulvers liegt dann bei 400° C bei 50. $10^{-7}/°$ C. Mit diesem Vorstufenpulver wurde analog Beispiel 1 ein Schalenkatalysator präpariert. Bei den beiden Drehrohrprozessen, der Porenbildner-Zersetzung und der Temperung ergibt sich ein starker Abrieb (ca. 5 Gew.%). Die Schale ist stark rissig und teilweise sind Stücke abgeplatzt. Für diesen Katalysator, bei dem der Wärmeausdehnungskoeffizient des Vorstufenpulvers nicht durch eine geeignete Temperaturbehandlung auf den des Trägers abgestimmt worden war, beträgt der Abrieb im La-Roche-Friabilator 15 Gew.%.

**Vergleichsbeispiel 2**

Das gemäß Beispiel 1 hergestellte Vorstufenpulver wird zur Präparation eines Katalysators im Dragierkessel verwendet. Dazu werden im 50 kg-Dragierkessel 30 kg des im Beispiel 1 verwendeten Steatit-Trägers vorgelegt und durch eine Rotation des Kessels von 21 Umdrehungen pro Minute bei 20° Kesselneigung in Mischbewegung gebracht. 31 kg des Vorstufenpulvers werden mit 2,5 kg D-Glucose und 1,5 kg Pentaerithrit in 22 l Wasser suspendiert. Die Oberfläche der bewegten Schüttung wird mit 200 m³/Stunde auf 90° C erhitzter Luft beaufschlagt. Zum Aufziehen der Schale wurde bei leicht abfallendem Durchfluß von anfänglich 0,5 l Suspension/Monat auf 0,48 l/Min. nach einer Stunde die Suspension durch eine Zweistoffdüse aufgesprüht. Nach Ende des Auftrags (ca. 80 Minuten) wird die Schale noch 10 Minuten bei weiterlaufendem Kessel verdichtet. Der erhaltene Schalenkatalysator wird 15 Stunden bei 40° C getrocknet, bei 400° C im Orehrohr der Porenbildner zersetzt und bei 550°C und 15 Minuten Verweilzeit im Drehrohr getempert.

Der erhaltene Katalysator hat die folgenden physikalischen Eigenschaften:

Der mittlere Durchmesser beträgt 5,3 mm mit einer Standardabweichung von 0,68 mm.

Der Roll- und Fallverschleiß im Roche-Friabilator (20 Umdrehungen/Min., 7 Minuten Laufzeit) liegt bei 1 Gew.% vor und bei 1,2 Gew.% nach einer Temperaturwechselbelastung zwischen 250 und 400° C (100 Zyklen in 50 Stunden). Beim Falltest von 100 ml durch ein 3,4 m langes Rohr mit 20 mm Innendurchmesser entstand ein Anteil an Bruch von < 2 mm von 0,2 Gew.%.

**Beispiel 2**

Die katalytische Wirkung des in Beispiel 1 hergestellten Katalysators wurde in einem technischen Reaktor-Rohr von 20,5 mm Innendurchmesser, das von außen durch ein Salzbad gekühlt wird, bei einer Katalysatorschüttungslänge von 2,7 m anhand der Umsetzung von Propen zu Acrolein getestet.

a) Bei einer Einspeisung von 5 Mol Propen/Stunde, 40 Mol Luft/Stunde und 10,1 Mol $H_2O$/Stunde werden bei einer Salzbadtemperatur von 351° C ein Umsatz von 94 %, eine Acrolein-Einsatzausbeute von 79,2 und eine Summenselektivität für Acrolein und Acrylsäure von 92,5 % erreicht.

b) Bei einer Einspeisung von 5 Mol Propen/Stunde, 38 Mol Luft/Stunde und 29 Mol rückgeführtem Abgas/Stunde (Zusammensetzung: 7 % $O_2$, 1 % Propen, 92 % Inertgas (Propan, Stickstoff, Kohlenstoffdioxid und Wasser)) werden bei einer Salzbadtemperatur von 355° C ein Umsatz von 94,9 %, eine Acrolein-Ausbeute von 79,5 % und eine Selektivität für Acrolein und Acrylsäure von 92 erhalten.

**Beispiel 3**

Als Vorstufe des katalytisch aktiven Materials wurde ein Rohkatalysatorpulver entsprechend DE-C 21 45 851 eingesetzt. Dieses wurde 8 Stunden bei 300° C einer Wärmebehandlung unterworfen. Es wies die Zusammensetzung 67,1 Gew.% $MoO_3$, 12,8 Gew.% $Fe_2O_3$ und 20,1 Gew.% $TiO_2$ auf. Der Hauptbereich (90 %) der Kornverteilung lag zwischen 1 und 10μm, mit dem 50 %-Wert bei 1,7μm. Die Wärmeleitfähigkeit des katalytischen Materials betrug 73. 10 $^{-7}$/° C.

2 kg dieses Pulvers wurden in 2 kg Wasser nach Zugabe von 0,12 kg Harnstoff (als Bindemittel) suspendiert. Als Träger für dieses Vorstufenmaterial wurden 6 kg Aluminiumsilikatträger mit einer spezifischen Oberfläche von kleiner 1 m²/g, einer Makroporosität, bei der 90 % der Poren zwischen 30 und 250μm liegen, einer oberflächlichen Rauhigkeit nach DIN 4768/1 mit einem Mittenrauhwert von 40μm, einem Durchmesser von 48 mm und einem Wärmeausdehnungskoeffizienten von 69. 10-7/° C (bei 400° C) in einen Driacoater 500 gegeben. Durch Einblasen von 4 m³/Min. auf 95°C vorgeheizter Luft und Drehen der Trommel mit 20 Umdrehungen/Min. wurde der Träger in intensive Misch- und Fließbewegung versetzt.

Die Suspension der Vorstufe wurde innerhalb von 75 Minuten so auf den fluidisierten Träger aufgesprüht, daß die Ablufttemperatur von anfangs 50 auf 44° C am Ende des Auftrags sank. Nach einer Nachverdichtungsphase von 5 Minuten im Driacoater bei weiterer Fluidisierung und Trocknung des Roh-Schalenkatalysators wurde dieser ca. 15 Stunden luftgetrocknet und dann abschließend 5 Stunden bei 425° C im Luftstrom getempert. Die Abriebfestigkeit im La-Roche-Friabilstor-Standardtest (7 Min., 20 U/min.) betrug 0,3 Gew.%.

5.180 g (ca. 3.760 ml) des fertigen Schalenkatalysators wurden in neun Rohre (Innendurchmesser 18,1 mm) eines Rohrbündelreaktors gleichmäßig eingefüllt. Die Schüttlänge am Katalysator betrug in den z. T. mit seitlich eingeführten Temperaturmessfühlern ausgerüsteten Rohren ca. 173 cm.

Der Rohrbündelreaktor wurde durch einen Kreislaufstrom von geschmolzenem Salz gekühlt. Die Salzbadtemperatur betrug 301° C. In den Reaktor wurde ein auf ca. 290 - 300° C vorgewärmter Gasstrom von 4.640 Nl/h folgender Zusammensetzung eingespeist: 11,1 Vol % Methanol, 12,8 Vol % Sauerstoff, Rest Inerte, im wesentlichen Stickstoff, neben geringen Mengen Wasserdampf (ca. 0,5 Vol %). Die Maximaltemperatur im Katalysatorbett betrug 355° C. Das den Reaktor verlassende Gas wurde sofort abgekühlt; die kondensierbaren Produkte wurden anschließend mit Wasser absorbiert. Über einen Bilanzierungszeitraum von 72 Stunden wurde bei einem Umsatz von 99 % des eingesetzten Methanols eine Ausbeute von 93,1 % Formaldehyd, bezogen auf eingesetztes Methanol, erhalten.

**Beispiel 4**

Entsprechend Beispiel 1 der DE-C 20 09 172 wurde ein Vorstufenpulver hergestellt, das Antimon, Molybdän, Vanadium und Wolfram im molaren Verhältnis von 6: 12: 3: 1,2 enthielt. Das walzengetrocknete Kopräzipitat wurde bei 250° C durch Calcinierung im Drehrohr weitgehend in die Oxide überführt und dann gemahlen. Das Pulver hatte dann eine Kornverteilung mit einem Hauptbereich (> 90 %) von 2 - 50μm mit Maximum bei 4,7μm und einen Wärmeausdehnungskoeffizienten bei 400° C von 86. $10^{-7}$/° C.

6,5 kg dieses Vorstufenpulvers wurden in 3,5 kg Wasser mit 0,2 kg Glucose als Bindemittel suspendiert und im Driacoater auf 6 kg Steatit-Träger (wie in Beispiel 1) innerhalb von 15 Min. aufgesprüht.

Dabei wurde der Träger durch auf 80° C vorgewärmte Luft und Rotation des Kessels in intensive Fließ- und Mischbewegung versetzt. Die aktivierende, abschließende Wärmebehandlung erfolgte bei 360° C im Drehrohr mit einer Verweilzeit von 15 Minuten. Die Abriebfestigkeit im La-Roche-Friabilator-Standardtest (7 Min. 20 U/Min.) betrug 0,05 Gew.%.

58 g dieses Schalenkatalysators wurden in ein Reaktorrohr von 16 m Innendurchmesser eingefüllt, das von außen durch eine Salzschmelze gekühlt wird. Es wurde bei 301° C Salzbadtemperatur ein Gasstrom, bestehend aus 1,4 Mol/h Luft, 0,5 Mol/h Wasser und 0,16 Mol/h Acrolein über den Katalysator geleitet und man erhielt einen Umsatz von 98,8 % und eine Acrylsäureausbeute von 94,5 %, bezogen auf eingesetztes Acrolein.

**Beispiel 5**

Ein Vorstufenpulver wurde gemäß DE-C 20 09 172 durch Kopräzipitation von 23,3 kg Antimontrioxid, 4,7 kg Ammonium metavanadat, 12,8 kg Titandioxid, 11,7 kg Montmorillonit und 5,8 kg pyrogene Kieselsäure, Walzentrocknung und 0,3 Stunden Wärmebehandlung bei 450° C hergestellt. Das erhaltene Pulver hat nach Mahlen ein Kornspektrum von 1 - 120μm (90 %) mit einem Maximum von 15 μm und einen Wärmeausdehnungskoeffizienten bei 400° C von 65. $10^{-7}$/° C.

9 kg dieses Vorstufenpulvers wurden in 6 kg Wasser mit 0 kg Glucose als Bindemittel und 0,6 kg Pentaerithrit suspendiert und im Driacoater auf 6 kg Aluminiumsilikatkugeln (wie Beispiel 3) in 85 Minuten aufgesprüht. Dabei wurde der Träger durch auf 80° C vorgeheizte Luft und Drehen des Kessels fluidisiert und der Aufsprühvorgang so gesteuert daß die Ablufttemperatur aus dem Kessel von anfangs 47° C auf 37° C am Ende abgesenkt wurde.

Nach Lufttrocknung (15 Stunden) wurde der Schalenkatalysator abschließend wärmebehandelt, indem er nacheinander 3 Stunden bei 550° C, 1 Stunde bei 650° C und 3 Stunden bei 770° C im Muffelofen getempert wurde. Der Abrieb des fertigen Schalenkatalysators betrug im La-Roche-Friabilator-Standardtest 0,1 Gew.%.

Der Katalysator ist vorzüglich geeignet für die Ammonoxidation von aromatischen und heteroaromatischen Kohlenwasserstoffen.

**Beispiel 6**

2 kg des in Beispiel 1 hergestellten Vorstufenpulvers werden in 1,9 kg Wasser unter Zusatz von 0,05 kg Glucose als Bindemittel suspendiert. Im Driacoater 500 werden 6 kg eines Aluminiumsilikatträgers mit einer spezifischen Oberfläche von kleiner 1 m²/g, einem Durchmesser von 4,8 mm, einer Makroporosität, bei der 90 % der Poren zwischen 70 und 500μm liegen, einer oberflächlichen Rauhigkeit nach DIN 4768/1 mit einem Mittenrauhwert von 48μm und einem Wärmeausdehnungskoeffizienten bei 400° C von 72. $10^{-7}$/° C durch Einblasen von 2 m³/min auf 70° C vorgeheizter Luft und durch Drehen der Trommel bei 12 Umdrehungen/Min. in intensive Misch- und Fließbewegung versetzt und auf den so bewegten Träger in 35 Minuten die Suspension analog zu Beispiel 1 so aufgesprüht, daß die Ablufttemperatur von anfänglich 43° C auf 38° C absinkt. Nach Trocknen des Rohkatalysators wird er bei 575° C im Drehrohr aktiviert. Der Abrieb, gemessen im La-Roche-Friabilator betrug 0,2 Gew.%.

**Beispiel 7**

Ein Vorstufenpulver wurde entsprechend Beispiel 1 hergestellt, nur mit dem Unterschied, daß der Samariumoxidlösung zusätzlich 0,4 kg Kaliumnitrat zugesetzt wurden. Das bei 470° C im Drehrohr calcinierte Vorstufenpulver hat einen Wärmeausdehnungskoeffizienten von 80. $10^{-7}$/° C.

9 kg dieses Vorstufenmaterials wurden mit 0,7 kg Pentaerithrit (Porenbildner) und 0,8 kg Glucose (Bindemittel) in 5,3 kg Wasser suspendiert und im Driacoater auf 6 kg intensiv bewegten Steatit-Trägern (wie in Beispiel I) aufgesprücht. Dabei wurden die 2,5 m3/min Zuluft auf 85° C vorgewärmt und die innerhalb von 95 Minuten aufgesprühte Suspension so dosiert, daß die Ablufttemperatur von anfänglich 51° C auf 42° C absank. Nach Trocknen, Zersetzen von Forenbildner und 8indemittel bei 400° C und Aktivieren bei 550° C im Drehrohr hatte der Katalysator einen Abrieb im La-Roche-Friabilator von 0,3 Gew.%.

## Beispiel 8

Ein Vorstufenpulver wurde analog zu Beispiel 1 hergestellt, indem einer Lösung von 6,7 kg Nickelnitrat $Ni(NO_3)_2$. $6H_2O$ 12,3 kg Kobaltnitrat $Co(NO_3)_2$. $6H_2O$ und 6,9 kg Eisennitrat $Fe_2(NO_3)_3$. $9H_2O$ in 30,4 kg Wasser nacheinander eine Lösung von 18,4 kg Ammoniummolybdat $(NH_4)_6Mo_7O_{24}$. $4H_2O$ in 24,1 kg 3,1 %iger Phosphorsäure, eine Lösung von 7 kg Bismutnitrat $Bi(NO_3)_3$. $5H_2O$ in 7,0 kg 0,8 %iger Salpetersäure und 6 kg pyrogene Kieselsäure (Aerosil® 200) unter Rühren zugesetzt wurde. Das entstehende Kopräzipitat wurde auf einem Walzentrockner bei 140° C getrocknet und bei 535° C im Drehrohr calciniert und dann in einer Stiftmühle gemahlen. Das erhaltene Pulver hat eine Kornverteilung von 5 - 80 µm (90 % Wert) mit einem Maximum bei 30 µm und einem Wärmeausdehnungskoeffizienten von 85. $10^{-7}$/° C.

Aus 7,5 kg dieses Vorstufenpulvers mit einem Zusatz von 0,6 kg Glucose und 0,5 kg Pentaerithrit in 6,2 kg Wasser und 6 kg Steatit-Träger wurde entsprechend Beispiel 1 ein abriebfester Schalenkatalysator im Driacoater 500 hergestellt. Der Abrieb im La-Roche-Friabilator betrug 0,25 Gew.%.

## Beispiel 9

Ein Vorstufenpulver wurde wie in Beispiel 8 hergestellt, nur mit einer zusätzlichen Beigabe von 0,2 kg $KNO_3$ zur ersten Lösung. Das erhaltene Pulver hat eine Kornverteilung von 3 - 70µm (90 % Wert) mit einem Maximum bei 25µm und einem Wärmeausdehnungskoeffizienten von 84. $10^{-7}$/° C.

Aus 5,5 kg dieses Vorstufenpulvers mit 0,4 kg Glucose in 4,5 kg Wasser und 6 kg Steatit-Träger wurde entsprechend Beispiel 1 ein abriebfester Schalenkatalysator im Driacoater 500 hergestellt. Der Abrieb im La-Roche-Friabilator betrug 0,3 Gew.%.

## Beispiel 10

50 ml des in Beispiel 8 hergestellten Katalysators wurden in einen von außen durch ein Salzbad auf 362° C temperierten Rohrreaktor von 16 mm Innendurchmesser gefüllt. Bei einer Einspeisung pro Stunde von 0,25 Mol Propen, 45 Nl Luft und 9,5 g Wasser erhält man einen Umsatz von 92,5 %, eine Acroleinausbeute, bezogen auf eingesetztes-Propen, von 80,5 % und eine Summenselektivität, bezogen auf eingesetztes Propen, von 95,8 %.

## Beispiel 11

50 ml des im Beispiel 6 hergestellten Katalysators wurden in einen von außen durch ein Salzbad auf 370° C temperierten Reaktor von 16 mm Innendurchmesser gefüllt. Bei einer Einspeisung pro Stunde von 0,15 Mol Isobuten, 35 Nl Luft und 10,5 kg Wasser erhält man einen Umsatz von 91 % und, bezogen auf eingespeistes Isobuten, eine Methacroleinausbeute von 74,1 % sowie eine Summenausbeute für Methacrolein und Methacrylsäure von 82,4 %.

## Beispiel 12

50 ml des im Beispiel 7 hergestellten Katalysators wurden in einen von außen durch ein Salzbad auf 355° C temperierten Reaktor von 16 mm Innendurchmesser gefüllt. Bei einer Einspeisung pro Stunde von 0,15 Mol t-Butanol, 35 Nl Luft und 10,5 kg Wasser erhält man einen Umsatz von 92,8 % und, bezogen auf eingespeistes t-Butanol, eine Methacroleinausbeute von 75,2 % und eine Summenausbeute für Methacrolein und Methacrylsäure von 81,9 %.

## Beispiel 13

50 ml des im Beispiel 9 hergestellten Katalysators wurden wie in Beispiel II bei einer Salzbadtemperatur von 382° C getestet. Der Umsatz lag bei 93,6 %, die auf eingespeistes Isobuten bezogene Methacroleinausbeute bei 75,6 % und die Summenselektivität für Methacrolein und Methacrylsäure bei 82,9 %.

## Beispiel 14

80 ml des nach Beispiel 5 hergestellten Katalysators wurden in einen von außen auf 430° C temperierten Reaktor von 20,5 mm Innendurchmesser gefüllt. Bei einer Einspeisung pro Stunde von 0,12 Mol β-Picolin, 80,5 Nl Luft, 16

Nl Ammoniak und 19 g Wasser erhielt man einen Umsatz von 89,5 % und eine auf eingesetztes β-Picolin bezogene Selektivität zu Nicotinsäurenitril von 79 %.

## Patentansprüche

1. Verfahren zur Herstellung von abriebfesten Schalenkatalysatoren aus einem oberflächlich rauhen, inerten Träger mit einer Korngröße von 0,5 bis 6 mm und einer diesen umhüllenden und in ihm verankerten Schale aus aktivem Katalysatormaterial durch mechanisch bewirktes mischendes Bewegen einer Schüttung des Trägers und Aufsprühen einer Suspension des Ausgangsmaterials für die Schale unter Teilabzug des Suspensionsmittels durch einen Gasstrom von 20 - 250°C, wobei eine im wesentlichen gleichbleibende Restfeuchte der Schale aufrechterhalten wird sowie Trocknen und Tempern, dadurch gekennzeichnet, daß man die bewegte Träger-schüttung gleichzeitig durch Einblasen eines fluidisierenden, die Vermischung verstärkenden Gasstromes von unten her auflockert, dieser Schüttung im Gegenstrom zum Gas die ein Bindemittel und gegebenenfalls Porenbildner enthaltende Suspension einer Vorstufe des katalytisch aktiven Materials in einer mit anwachsender Dicke der Schale zunehmenden Menge zuführt, wobei die Mengen von abgezogenem und aufgesprühtem Suspensionsmittel in einem weitgehend konstanten Verhältnis gehalten werden, das von der jeweils verwendeten Kombination aus Träger und Vorstufe bestimmt wird und wobei die Wärmeausdehnungskoeffizienten von Träger und getrockneter pulverförmiger Vorstufe so abgestimmt sind, daß sie sich im Höchstfall um 15 % unterscheiden, daß man nach Beendigung des Aufsprühens die Schale durch Fortsetzen der verstärkten Mischbewegung verdichtet, dann die mechanische Mischbewegung einstellt, das Gut im weiterströmenden Gas trocknet und es schließlich, gegebenenfalls nach Zersetzen eines zugegebenen Porenbildners, tempert.

2. Verfahren nach Anspruch 1, dadurch gekennzeichnet, daß man das Aufbringen und Trocknen der Schale in einer Vorrichtung durchführt, die primär aus einer zylindrisch bzw. kegelig geformten und horizontal montierten Trommel besteht, indem über im Außenmantel der Trommel angebrachte Luftkanäle und über auf der Trommelinnenwand angeordnete, auf der Drehrichtungs-abgekehrten Seite perforierte Hohlrippen Trockenluft ausschließlich von der Gutbett-Unterseite her eingebracht wird.

3. Verfahren nach Anspruch 1 oder 2, dadurch gekennzeichnet, daß man den fluidisierenden Gasstrom suf einen spezifischen Durchsatz von 15 - 50 Nm$^3$/Stunde/Liter Träger einstellt.

4. Verfahren nach den Ansprüchen 1 - 3, dadurch gekennzeichnet, daß als Suspensionsmittel Wasser verwendet wird.

5. Verfahren nach den Ansprüchen 1 - 4, dadurch gekennzeichnet, daß die Suspension zu 20 bis 80, vorzugsweise 40 - 70, insbesondere 55 Gew.% aus der pulverförmigen Vorstufe besteht.

6. Verfahren nach den Ansprüchen 1 - 5, dadurch gekennzeichnet, daß die Suspension als Bindemittel 0,5 - 10, vorzugsweise 2 - 5, insbesondere um 4 Gew.% Glucose oder Harnstoff enthält.

7. Verfahren nach den Ansprüchen 1 - 6, dadurch gekennzeichnet, daß der Suspension des pulverförmigen Ausgangsmaterials für die Schale 1 - 10 Gew.%, bezogen auf das Gewicht dieses Materials, eines im Suspensionsmittel schwerlöslichen feinteiligen Porenbildners zugesetzt wird, welcher unterhalb der Tempertemperatur durch Thermolyse oder Oxidation entfernbar ist.

8. Verfahren nach den Ansprüchen 1 - 7, dadurch gekennzeichnet, daß als Vorstufe des katalytisch aktiven Materials ein getrocknetes oder unterhalb der Tempertemperatur calciniertes Kopräzipitat aus vereinigten Salzlösungen der katalytisch aktiven Elemente eingesetzt wird.

9. Verfahren nach den Ansprüchen 1 - 8, dadurch gekennzeichnet, daß die Vorstufe ein Pulver mit einer Kornverteilung von 1 - 150 μm mit einem vorzugsweisen Maximum im Bereich von 1,5 - 30 um ist.

10. Verfahren nach den Ansprüchen 1 - 9, dadurch gekennzeichnet, daß die Menge der pulverförmigen Vorstufe das 0,1 - 2-fache des Trägergewichts beträgt.

11. Verfahren nach den Ansprüchen 1 - 10, dadurch gekennzeichnet, daß der Wärmeausdehnungskoeffizient des Vorstufenpulvers durch eine Temperaturvorbehandlung bei 250 - 600° C auf den des Trägers abgestimmt wird.

12. Verfahren nach den Ansprüchen 1 - 11, dadurch gekennzeichnet, daß als Vorstufe ein oxidisches Pulver der Zusammensetzung $Ni_aCo_bFe_cBi_dP_eMo_fO_x$, in dem a eine Zahl von 2 - 20, b eine Zahl von 0 - 15, a und b eine Zahl von 2 - 20, c eine Zahl von 0,1 - 7, d eine Zahl von 0,1 - 4, e eine Zahl von 0,1 - 4, f etwa 12 und x eine Zahl von 35 - 85 ist sowie zusätzlich 0,2 bis 5 % Tantal oder Samarium, berechnet als $Ta_2O_5$ oder $Sm_2O_3$ sowie gegebenenfalls noch 0,05 bis 3,0 % Alkali- oder Erdalkalimetall, berechnet als Oxid, gegebenenfalls auf einer Trägersubstanz aus einem Schichtsilikat und/oder hochdispersem Siliciumdioxid -im ersten Fall im Gewichtsverhältnis 10: 1 bis 1: I- eingesetzt und der Schalenkatalysator 0,05 - 5 Stunden bei 520 - 650° C getempert wird.

13. Verfahren nach den Ansprüchen 1 - 11, dadurch gekennzeichnet, daß als Vorstufe ein oxidisches Pulver der Zusammensetzung $Sb_{1-60}Mo_{12}V_{0,1-12}M_{0-12}O_x$, wobei M mindestens eines der Elemente Blei, Silber, Kupfer, Zinn, Titan, Wismut bedeutet, eingesetzt und der Schalenkatalysator 0,05 - 5 Stunden bei 320 - 450° C getempert wird.

14. Verfahren nach den Ansprüchen 1 - 11, dadurch gekennzeichnet, daß als Vorstufe ein oxidisches Pulver aus Molybdän und Eisen, mit einem MoO₃: Fe₂O₃-Verhältnis von 10, dem gegebenenfalls 3 - 60 Gew.% TiO₂ beigemengt sein können, eingesetzt und der Schalenkatalysator 3 - 10 Stunden bei 300 - 500° C getempert wird.

15. Verfahren nach den Ansprüchen 1 - 11, dadurch gekennzeichnet, daß als Vorstufe ein oxidisches Pulver aus den Oxiden von Antimon und Vanadin im Verhältnis 1,1: 1 bis 50: 1, das zusätzlich wenigstens eines der Elemente Eisen, Kupfer, Titan, Kobalt, Mangan und Nickel und gegebenenfslls Trägersubstanz aus Schichtensilikat und hochdispersem Siliciumdioxid enthält, eingesetzt und der erhaltene Schalenkatalysator 2 - 8 Stunden zwischen 600 und 1.100° C getempert wird.

16. Verfahren nach den Ansprüchen 1 - 14, dadurch gekennzeichnet, daß als Träger α-Aluminiumoxid, Aluminiumsilikat, Magnesiumsilikat oder Siliciumcarbid eingesetzt wird.

17. Verfahren nach Anspruch 16, dadurch gekennzeichnet, daß das α-Aluminiumoxid bzw. das Aluminiumsilikat eine Porosität aufweist, gemäß welcher 90 % der Poren im Bereich von 2 - 2000, bevorzugt 20 - 300μm liegen.

18. Verfahren nach Anspruch 16, dadurch gekennzeichnet, daß das Magnesiumsilikat bzw. das Siliciumcarbid praktisch porenfrei ist.

19. Verfahren nach den Ansprüchen 16 - 18, dadurch gekennzeichnet, daß die Rauhigkeit der Trägeroberfläche einen Mittenrauhwert von 5 - 50μm nach DIN 4768/1, gemessen mit dem Rauhtiefenmesser nach Hommel, hat.

20. Verwendung des nach Anspruch 13 sowie Ansprüchen 16-19 erhältlichen Schalenkatalysators zur Herstellung von Acrylsäure oder Methacrylsäure durch Oxidation von Acrolein oder Methacrolein.

21. Verwendung des nach Anspruch 14 sowie Ansprüchen 16-19 erhältlichen Schalenkatalysators zur Herstellung von Formaldehyd durch Oxidation von Methanol.

22. Verwendung des nach den Ansprüchen 15 - 19 erhältlichen Schalenkatalysators zur Herstellung von aromatischen und heteroaromatischen Nitrilen durch Ammonoxidation von alkylsubstituierten aromatischen und heteroaromatischen Kohlenwasserstoffen.

## Claims

1. A process for the production of abrasion-resistant shell catalysts consisting of a rough-surfaced inert carrier having a particle size of from 0.5 to 6 mm and a shell of active catalyst material which surrounds it and is fixed in it, by mechanically effected mixing movement of a mass of the carrier and spraying of a suspension of the starting material for the shell with partial removal of the suspension agent by a gas stream at from 20 to 250°C so as to maintain a substantially constant residual moisture of the shell, as well as drying and tempering, characterised in that the moved mass of carrier is simultaneously loosened from below by blowing in a fluidizing gas stream which enhances the mixing, the suspension of a precursor of the catalytically active material containing a binder and optionally pore-forming agent is supplied to this mass in a counter-current to the gas in a quantity which increases as the thickness of the shell increases, the quantities of removed and sprayed suspension agent being kept in a substantially constant ratio which is determined by the respectively used combination of carrier and precursor, and the coefficients of thermal expansion of carrier and dried powdered precursor being adapted such that they differ at most by 15%, in that, on termination of the spraying operation, the shell is compacted by continuing the enhanced mixing movement, then the mechanical mixing movement is terminated the product is dried in the still-flowing gas and is finally tempered optionally after decomposition of an added pore-forming agent.

2. A process according to claim 1, characterised in that the application and drying of the shell are carried out in an apparatus primarily comprising a cylindrically or conically shaped and horizontally mounted drum, into which dry air is introduced exclusively from the underside of the bed of product via air ducts arranged in the outer jacket of the drum and via hollow ridges which are arranged on the internal wall of the drum and are perforated on the side turned away from the rotational direction.

3. A process according to claim 1 or 2, characterised in that the fluidizing gas stream is adjusted to a specific throughput of from 15 to 50 Nm³/hour/litre of carrier.

4. A process according to claims 1 to 3, characterised in that water is used as suspension agent.

5. A process according to claims 1 to 4, characterised in that the suspension is composed of from 20 to 80, preferably from 40 to 70, in particular of 55% by weight of the powdered precursor.

6. A process according to claims 1 to 5, characterised in that the suspension contains from 0.5 to 10, preferably from 2 to 5, in particular about 4% by weight of glucose or urea as binder.

7. A process according to claims 1 to 6, characterised in that from 1 to 10% by weight, based on the weight of this material, of a finely divided pore-forming agent which is sparingly soluble in the suspension agent is added to the suspension of the powdered starting material for the shell, the pore-forming agent being removable by thermolysis or oxidation below the tempering temperature.

8. A process according to claims 1 to 7 characterised in that a co-precipitate of combined salt solutions of the catalytically active elements, which is dry or is calcined below the tempering temperature, is used as precursor of the catalytically active material.

9. A process according to claims 1 to 8, characterised in that the precursor is a powder having a particle distribution of from 1 to 150 μm with a preferred maximum in the range of from 1.5 to 30 μm.

10. A process according to claims 1 to 9, characterised in that the quantity of the powdered precursor is from 0.1 to 2 times the carrier weight.

11. A process according to claims 1 to 10, characterised in that the coefficient of thermal expansion of the precursor powder is adapted to that of the carrier by a thermal pretreatment at from 250 to 600°C.

12. A process according to claims 1 to 11, characterised in that, as precursor, there is used an oxidic powder having the composition $Ni_aCo_bFe_cBi_dP_eMo_fO_x$, in which $a$ is a number from 2 to 20, $b$ is a number from 0 to 15, $a$ and $b$ are a number from 2 to 20, $c$ is a number from 0.1 to 7, $d$ is a number from 0.1 to 4, $e$ is a number from 0.1 to 4, $f$ is approximately 12 and $x$ is a number from 35 to 85, and also 0.2 to 5% of tantalum or samarium, calculated as $Ta_2O_5$ or $Sm_2O_3$ and optionally a further 0.05 to 3.0 % of alkali or alkaline earth metal calculated as oxide, optionally on a carrier substance composed of a layered silicate and/or highly dispersed silicon dioxide - in the first case in a ratio by weight of from 10:1 to 1:1, and the shell catalyst is tempered for from 0.05 to 5 hours at from 520 to 650°C.

13. A process according to claims 1 to 11, characterised in that, as precursor there is used an oxidic powder having the composition $Sb_{1-60}Mo_{12}V_{0.5-25}W_{0.1-12}M_{0-12}O_x$, wherein M represents at least one of the elements lead, silver, copper, tin, titanium and bismuth, and the shell catalyst is tempered for from 0.05 to 5 hours at from 320 to 450°C.

14. A process according to claims 1 to 11, characterised in that, as precursor, there is used an oxidic powder composed of molybdenum and iron having an $MoO_3$: $Fe_2O_3$-ratio of 10, to which from 3 to 60% by weight of $TiO_2$ may optionally be added, and the shell catalyst is tempered for from 3 to 10 hours at from 300 to 500°C.

15. A process according to claims 1 to 11, characterised in that, as precursor, there is used an oxidic powder composed of the oxides of antimony and vanadium in a ratio of from 1.1:1 to 50:1 which additionally contains at least one of the elements iron, copper, titanium, cobalt, manganese and nickel and optionally carrier substance composed of layered silicate and highly dispersed silicon dioxide, and the shell catalyst obtained is tempered for from 2 to 8 hours at betwéen 600 and 1100°C.

16. A process according to claims 1 to 14 characterised in that α-aluminium oxide aluminium silicate, magnesium silicate or silicon carbide is used as carrier.

17. A process according to claim 16, characterised in that the α-aluminium oxide or the aluminiun silicate has a porosity according to which 90% of the pores lie in the range of from 2 to 2000, preferably from 20 to 300 μm.

18. A process according to claim 16, characterised in that the magnesium silicate or the silicon carbide are substantially pore-free.

19. A process according to claims 16 to 18, characterised in that the roughness of the carrier surface has a mean roughness value of from 5 to 50 μm according to DIN 4768/1, measured using the Hommel roughness measuring device.

20. Use of the shell catalyst obtainable according to claim 13 and claims 16 to 19 for the production of acrylic acid or methacrylic acid by oxidation of acrolein or methacrolein.

21. Use of the shell catalyst obtainable according to claim 14 and claims 16 to 19 for the production of formaldehyde by oxidation of methanol.

22. Use of the shell catalyst obtainable according to claims 15 to 19 for the production of aromatic and heteroaromatic nitriles by ammonoxidation of alkyl-substituted aromatic and heteroaromatic hydrocarbons.

**Revendications**

1°) Procédé pour la préparation de catalyseurs en coquilles résistant à l'abrasion constitués par un support inerte à surface rugueuse avec une grosseur de grain de 0,5 à 6 mm, et une coque qui l'enrobe et qui est fixée sur celui-ci en une matière catalytiquement active, par lequel on communique un mouvement, de mélange, produit mécaniquement à une masse de supports, et on pulvérise une suspension de la matière destinée à constituer la coque avec élimination partielle de l'agent de mise en suspension au moyen d'un courant gazeux à 20 à 250°C, une humidité résiduelle de la coque essentiellement constante étant maintenue, ces opérations étant suivies d'un séchage et d'un traitement thermique, procédé caractérisé en ce que l'on ameublit par le bas la masse de supports en mouvement, en insufflant simultanément un courant gazeux fluidisant, intensifiant le mélange, et on introduit dans cette masse, a contre-courant par rapport au gaz, la suspension, contenant un liant, et éventuellement un formateur de pores, d'un précurseur de la matière catalytiquement active dans une proportion augmentant avec l'épaisseur de la coque, les quantités d'agent de mise en suspension extraites et pulvérisées étant maintenues dans un rapport largement constant, déterminé par la combinaison de supports et de précurseurs utilisée, les coefficients de dilatation thermique du support et du précurseur en poudre séché étant mis en accord de façon à ne différer, dans le cas maximum, que de 15 % et en ce que, après achèvement de la pulvérisation de la coque, on la compacte en poursuivant le mouvement de mélange renforcé, puis on arrête le mouvement mécanique de mélange, on sèche la matière en poursuivant le passage du gaz et finalement la soumet à un traitement thermique, éventuellement après avoir décomposé le formateur de pores incorpore.

2°) Procédé suivant la revendication 1, caractérisé en ce que l'on effectue l'application et le séchage de la coque dans un dispositif qui est constitué principalement d'un tambour de forme cylindrique ou conique, monté horizontalement, dans lequel l'air sec est introduit, exclusivement par la face inférieure du lit de produit, par l'intermédiaire de canaux d'air montés sur l'enveloppe extérieure du tambour et de nervures évidées, disposées sur la paroi intérieure du tambour et perforées sur la face qui est à l'opposé du sens de rotation.

3°) Procédé suivant les revendications 1 ou 2, caractérisé en ce que l'on ajuste le courant gazeux de fluidisation a un debit specifique de 15 à 50 Nm³/heure/litre de supports.

4°) Procédé suivant les revendications 1 à 3, caractérisé en ce que, l'on utilise l'eau en tant qu'agent de mise en suspension.

5°) Procédé suivant les revendications 1 à 4, caractérisé en ce que la suspension est constituée, pour 20 à 80, ou mieux pour 40 à 70, en particulier pour 55 % en poids, du précurseur en poudre.

6°) Procédé suivant les revendications 1 à 3, caractérisé en ce que la suspension contient, comme liant, 0,5 à 10, de préférence 2 à 5, en particulier environ 4 % en poids de glucose ou d'urée.

7°) Procédé suivant les revendications 1 à 6, caractérisé en ce que l'on ajoute à la suspension de la matière de départ pour la formation de la coque, 1 à 10 % en poids, calculé sur le poids de cette matière, d'un formateur de pores finement divisé, difficilement soluble dans l'agent de mise en suspension, et qui peut être éliminé par thermolyse ou oxydation en-dessous de la température du traitement thermique.

8°) Procédé suivant les revendications 1 à 7, caractérisé en ce que, comme précurseur de la matière catalytiquement active, on met en oeuvre un précipité mixte séché ou calciné à une température inférieure à celle du traitement thermique, de solutions salines réunies des éléments catalytiquement actifs.

9°) Procédé suivant les revendications 1 à 8, caractérisé en ce que le précurseur est une poudre avec une granulométrie de 1 à 150 μ, avec un maximum préférentiel dans la zone de 1,5 à 30 μ.

10°) Procédé suivant les revendications 1 à 9, caractérisé en ce que la quantité de precurseur en poudre se monte à 0,1 à 2 fois le poids des supports.

11°) Procédé suivant les revendications 1 à 10, caractérisé en ce que le coefficient de dilatation thermique du précurseur en poudre est adapté à celui du support au moyen d'un traitement thermique préalable, à 250 à 600°C.

12°) Procédé suivant les revendications 1 à 11, caractérisé en ce que, comme précurseur, on utilise une poudre d'oxydes présentant la composition $Ni_aCo_bFe_cBi_dP_eMo_fO_x$, dans laquelle a est un nombre de 2 à 20, b, un nombre de 0 à 15, a et b un nombre de 2 à 20, c, un nombre de 0,1 à 7, d, un nombre de 0,1 à 4, e, un nombre de 0,1 à 4, f, environ 12 et x, un nombre de 35 à 85, ainsi que, en plus 0,2 a 5 % de tantale ou de samarium, calculé en $Ta_2O_5$ ou $Sm_2O_3$, ainsi qu'éventuellement encore 0,05 à 3 % de métaux alcalins ou alcalino-terreux, calculé en oxydes, éventuellement sur une substance de support, faite d'un phyllosilicate, et/ou d'un dioxyde de silicium fortement dispersé, dans le premier cas, dans un rapport en poids de 10: 1 à 1: 1, le catalyseur subissant un traitement thermique pendant 0,05 a 5 heures à 520 à 650°C.

13°) Procédé suivant les revendications 1 à 11, caractérisé en ce que, comme précurseur, on utilise une poudre d'oxydes ayant la composition $Sb_{1\ à\ 60}Mo_{12}V_{0,5\ à\ 25}W_{0,1\ à\ 12}M_{0\ à\ 12}O_x$, dans laquelle M est au moins un des éléments plomb, argent, cuivre, étain, titane, bismuth, le catalyseur étant soumis à un traitement thermique pendant 0,05 à 5 h à 320 à 450°C.

14°) Procédé suivant les revendications 1 à 11, caractérisé en ce que, comme précurseur, on utilise une poudre d'oxydes de molybdène et de fer avec un rapport $MoO_3$: $Fe_2O_3$ de 10, à laquelle il peut être éventuellement ajouté 3 à 60 % en poids de $TiO_2$, le catalyseur étant soumis pendant 3 à 10 heures à un traitement thermique à 300 à 500°C.

15°) Procédé suivant les revendications 1 à 11, caractérisé en ce que, comme précurseur, on utilise une poudre constituée d'oxydes d'antimoine et de vanadium dans un rapport de 1,1: 1 à 50: 1 qui contient, en plus, au moins un des éléments fer, cuivre, titane, cobalt, manganèse et nickel, et éventuellement une substance support constituée de phyllosilicate et de dioxyde de silicium fortement dispersé, le catalyseur étant soumis à un traitement thermique pendant 2 à 8 heures entre 600 et 1 100°C.

16°) Procédé suivant les revendications 1 à 14, caractérisé en ce que l'on utilise comme supports les oxydes d'aluminium-α, silicate d'aluminium, silicate de magnésium ou carbure de silicium. 17°) Procédé suivant la revendication 16, caractérisé en ce que l'oxyde d'aluminium-α ou le silicate d'aluminium présente une porosité suivant laquelle 90 % des pores se situent dans un ordre de grandeur de 2 a 2000 μ, et de préférence de 20 à 300 μ.

18°) Procédé suivant la revendication 16, caractérisé en ce que le silicate de magnésium ou le carbure de silicium sont pratiquement exempts de pores.

19°) Procédé suivant les revendications 16 à 18, caractérisé en ce que la rugosité de la surface des supports présente une valeur moyenne de rugosité de 5 à 50 μ, suivant la norme DIN 4768/1, mesurée avec l'appareil de mesure de la profondeur de rugosité suivant Hommel.

20°) Utilisation du catalyseur obtenu suivant les revendications 13 ainsi que 16 à 19 pour la préparation d'acide acrylique ou méthacrylique par oxydation d'acroléine ou de méthacroléine.

21°) Utilisation du catalyseur obtenu suivant la revendication 14 ainsi que les revendications 16 à 19 pour la préparation de formol par oxydation du méthanol.

22°) Utilisation du catalyseur obtenu suivant les revendications 15 à 19 pour la préparation de nitriles aromatiques et hétéroaromatiques par ammonoxydation d'hydrocarbures aromatiques et hétéroaromatiques substitués par un alcoyl.